# EUROPEAN PATENT SPECIFICATION

(11) **EP 2 421 499 B1**
(45) Date of publication and mention of the grant of the patent: **06.03.2013**
(21) Application number: 10713460.3
(22) Date of filing: 13.04.2010
(51) Int. Cl.: A61K 8/29, A61K 8/362, A61K 8/365, A61Q 5/12

(54) **Hair styling method**
Haarstylingverfahren
Méthode de coiffage

(30) Priority: 24.04.2009 EP 09158726
(43) Date of publication of application: 29.02.2012
(73) Proprietor: Unilever PLC, London, Greater London EC4Y 0DY (GB); Unilever N.V., 3013 AL Rotterdam (NL)
(72) Inventor: PAUL, Prem, Kumar, Cheyalazhagan, Merseyside CH63 3JW (GB); PYE, Susan, Merseyside CH63 3JW (GB)
(74) Representative: Tansley, Sally Elizabeth
(86) International application number: PCT/EP2010/054818
(87) International publication number: WO 2010/121924

(56) References cited:
- EP-A- 0 586 235
- US-A- 3 857 727
- US-A1- 2004 131 566
- US-A1- 2004 266 978
- US-A1- 2005 214 238
- US-A1- 2006 045 862

## Description

The invention relates to hair styling methods.

Hair can be styled in many ways. One manner of styling hair is decrease its volume. Decreasing the volume is seen as a way of mitigating the frizzy appearance of the hair. However many products that decrease the volume of the hair leave it with negative sensory benefits in that it feets stiff and sticky.

A leave-on hair cosmetic composition comprising a citric acid or a salt-thereof is known from US 2005/021 4238 A1.

There remains the need for a method that can decrease the volume of hair and that leave it feeling soft, smooth and easy to comb.

The present invention relates to a method of treating hair comprising the step of applying to the hair a composition comprising from 0.001 wt% to 20 wt% of the total composition of titanium salt selected from the group consisting of citrate, oxalate and tartrate or the metal alkali salt of these and in which at least one of these is a sodium salt of titanium citrate.

Also described is the use of a titanium salt for detangling hair, decreasing the volume of hair, smoothing hair; making hair easier to comb and softening the hair

The level of titanium salt in the total composition is preferably from 0.01 to 10 wt% of the total composition, more preferably from 0.1 to 5 wt%.

The formulation may include conditioning materials such as surfactants, cationic conditioners suitable for hair, quaternary silicone polymers, silicone based conditioners and their emulsions, and amino functional silicones and their emulsions. Silicone based products are particularly preferred.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst. The viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use the invention will typically have an average silicone particle size in the composition of less than 30, preferably less than 20, more preferably less than 10 microns. Most preferably the average silicone particle size of the emulsified silicone in the composition is less than 2 microns, ideally it ranges from 0.01 to 1 micron. Silicone emulsions having an average silicone particle size of ≤ 0.15 microns are generally termed microemulsions.

Particle size may be measured by means of a laser light scattering technique, using a 2600D Particle Sizer from Malvern Instruments.

Suitable silicone emulsions for use in the invention are also commercially available in a pre-emulsified form.

Examples of suitable pre-formed emulsions include emulsions DC2-1766, DC2-1784, and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

A further preferred class of silicones for inclusion in the invention are amino functional silicones. By "amino functional silicone" is meant a silicone containing at least one primary, secondary or tertiary amine group, or a quaternary ammonium group.

Examples of suitable amino functional silicones include:
(i) polysiloxanes having the CTFA designation "amodimethicone", and the general formula:

   HO-[Si(CH₃)₂-O-]ₓ-[Si(OH)(CH₂CH₂CH₂-NH-CH₂CH₂NH ₂)-O-]_{y}-H

   in which x and y are numbers depending on the molecular weight of the polymer, generally such that the molecular weight is between about 5,000 and 500,000.
(ii) polysiloxanes having the general formula:

   R^{'}ₐG₃-ₐ-Si(OSiG₂)ₙ-(OSiG_{b}R^{'}_{2-b})ₘ-O-SiG₃₋ₐ-R^{'}ₐ

   in which:
   G is selected from H, phenyl, OH or C₁₋₈ alkyl, e.g. methyl;
   a is 0 or an integer from 1 to 3, preferably 0;
   b is 0 or 1, preferably 1;
   m and n are numbers such that (m + n) can range from 1 to 2000, preferably from 50 to 150;
   m is a number from 1 to 2000, preferably from 1 to 10;
   n is a number from 0 to 1999, preferably from 49 to 149, and
   R^{'} is a monovalent radical of formula -C_{q}H_{2q}L in which q is a number from 2 to 8 and L is an aminofunctional group selected from the following:

   -NR^{"}-CH₂-CH₂-N(R^{"})₂

   -N(R^{"})₂

   -N⁺(R^{"})₃A⁻

   -N⁺H(R^{"})₂ A⁻

   -N⁺H₂(R^{"}) A⁻

   -N(R^{"})-CH₂-CH₂-N⁺H₂(R^{"}) A⁻

   in which R^{"} is selected from H, phenyl, benzyl, or a saturated monovalent hydrocarbon radical, e.g. C₁₋₂₀ alkyl, and;
   A is a halide ion, e.g. chloride or bromide.
   Suitable amino functional silicones corresponding to the above formula include those polysiloxanes termed "trimethylsilylamodimethicone" as depicted below, and which are sufficiently water insoluble so as to be useful in compositions of the invention:

   Si(CH₃)₃ -O-[Si(CH₃)₂-O-]ₓ -[Si(CH₃)(R-NH-CH₂CH₂NH₂)-O-]y-Si (CH3)3

   wherein x + y is a number from about 50 to about 500, and wherein R is an alkylene group having from 2 to 5 carbon atoms. Preferably, the number x + y is in the range of from about 100 to about 300.
(iii) quaternary silicone polymers having the general formula:

   {(R¹)(R²)(R³) N⁺ CH₂CH(OH)CH₂O(CH₂)₃[Si(R⁴)(R⁵)-O-]ₙ-Si(R⁶)(R⁷)-(CH₂)₃-O-CH₂CH(OH)CH₂N⁺(R⁸)(R⁹)(R¹⁰)} (X⁻)₂

   wherein R¹ and R¹⁰ may be the same or different and may be independently selected from H, saturated or unsaturated long or short chain alk(en)yl, branched chain alk(en)yl and C₅-C₈ cyclic ring systems;
   R² thru' R⁹ may be the same or different and may be independently selected from H, straight or branched chain lower alk(en)yl, and C₅-C₈ cyclic ring systems; n is a number within the range of about 60 to about 120, preferably about 80, and
   X⁻ is preferably acetate, but may instead be for example halide, organic carboxylate, organic sulphonate or the like.

Suitable quaternary silicone polymers of this class are described in EP-A-0 530 974.

Amino functional silicones suitable for use in the invention will typically have a mole % amine functionality in the range of from about 0.1 to about 8.0 mole %, preferably from about 0.1 to about 5.0 mole %, most preferably from about 0.1 to about 2.0 mole %. In general the amine concentration should not exceed about 8.0 mole % since we have found that too high an amine concentration can be detrimental to total silicone deposition and therefore conditioning performance.

The viscosity of the amino functional silicone can suitably range from about 100 to about 500,000 cst.

Specific examples of amino functional silicones suitable for use in the invention are the aminosilicone oils DC2-8220, DC2-8166, DC2-8466, and DC2-8950-114, DC7134 (all ex Dow Corning), and GE 1149-75, (ex General Electric Silicones).

Also suitable are emulsions of amino functional silicone oils with non ionic and/or cationic surfactant.

Suitably such pre-formed emulsions will have an average amino functional silicone particle size in the shampoo composition of less than 30, preferably less than 20, more preferably less than 10 microns. Most preferably the average amino functional silicone particle size in the composition is less than 2 microns, ideally it ranges from 0.01 to 1 micron. Silicone emulsions having an average silicone particle size of ≤ 0.15 microns are generally termed microemulsions.

Pre-formed emulsions of amino functional silicone are also available from suppliers of silicone oils such as Dow Corning and General Electric. Specific examples include DC929 Cationic Emulsion, DC939 Cationic Emulsion, and the non-ionic emulsions DC2-7224, DC2-8467, DC2-8177 and DC2-8154 (all ex Dow Corning).

The total amount of silicone incorporated into compositions used according to the invention depends on the level of conditioning desired and the material used. A preferred amount is from 0.01 to about 10% by weight of the total composition.

We have found that a total amount of silicone of from 0.3 to 5%, preferably 1 to 8%, by weight of the total composition is a suitable level.

In some aspects of this invention it is desirable if the composition comprises a styling aid.

Particularly useful as styling aids with this invention are hair styling polymers. Hair styling polymers are well known and many such polymers are available commercially which contain moieties which render the polymers cationic, anionic, amphoteric or nonionic in nature. The polymers may be synthetic or naturally derived.

Compositions used according to the present invention are preferably formulated into hair care compositions with hair styling claims. The compositions are preferably used to non-permanently style human hair and, more preferably, they are packaged and labeled as such. The term non-permanently means that during the styling process the inter cystine-disulphide bonds are not broken. This means that preferably there are no reductive agents in the hair care composition. It is preferable if the composition is free of thioglycolic acid and thiolactic acid.

It is also preferable if the composition is free of metal complexes of porphyrines, naphtalocyanines, phtalocyanines, cyanobalamines and derivatives thereof.

It is preferred if the products are left on hair after application and not immediately washed off (within 10 minutes, preferably 60 minutes of application). Such products are known as leave in compositions. Leave in styling compositions are particularly preferred.

Preferred product forms are leave on formulations such as mousses, gels, waxes, sprays and aerosols. Particularly preferred are non-aerosol product forms, in particular creams.

Hair styling waxes, creams or gels also typically contain a structurant or thickener, typically in an amount of from 0.01 % to 10% by weight of the total composition, more preferably from 0.1 to 5 wt%

Examples of suitable structurants or thickeners are polymeric thickeners such as carboxyvinyl polymers. A carboxyvinyl polymer is an interpolymer of a monomeric mixture comprising a monomeric olefinically unsaturated carboxylic acid, and from about 0.01 % to about 10% by weight of the total monomers of a polyether of a polyhydric alcohol. Carboxyvinyl polymers are substantially insoluble in liquid, volatile organic hydrocarbons and are dimensionally stable on exposure to air. Suitably the molecular weight of the carboxyvinyl polymer is at least 750,000, preferably at least 1,250,000, most preferably at least 3,000,000. Preferred carboxyvinyl polymers are copolymers of acrylic acid cross-linked with allylsucrose or allylpentaerythritol as described in US Patent 2,798,053. These polymers are provided by B.F.Goodrich Company as, for example, CARBOPOL 934, 940, 941 and 980. Other materials that can also be used as structurants or thickeners include those that can impart a gel-like viscosity to the composition, such as water soluble or colloidally water soluble polymers like cellulose ethers (e.g. methylcellulose, hydroxyethylcellulose, hydroxypropylmethylcellulose and carboxymethylcellulose), guar gum, sodium alginate, gum arabic, xanthan gum, polyvinyl alcohol, polyvinyl pyrrolidone,hydroxypropyl guar gum, starch and starch derivatives, and other thickeners, viscosity modifiers, gelling agents, etc. Particularly preferred thickeners are those based on acrylate, such as sodium acrylate copolymer, a commercial example of this polymer is Tinovis CD Ex Ciba. It is also possible to use inorganic thickeners such as bentonite or laponite clays. Such styling products frequently include a carrier and further additional components. The carriers and additional components required to formulate such products vary with product type and can be routinely chosen by one skilled in the art. The following is a description of some of these carriers and additional components.

The pH of the formulation is preferably from 2.5 to 8 at 25°C. More preferably from 3 to 6.

Hair care compositions used according to the present invention can comprise a carrier, or a mixture of such carriers, which are suitable for application to the hair. The carriers are present at from about 0.5% to about 99.5%, preferably from about 5.0% to about 99.5%, more preferably from about 10.0% to about 98.0%, of the composition. As used herein, the phrase "suitable for application to hair" means that the carrier does not damage or negatively affect the aesthetics of hair or cause irritation to the underlying skin.

Compositions used according to the invention comprise a buffer or pH adjuster. Preferred buffers or pH adjusters include weak acids and bases such glycine/sodium hydroxide, citric acid, lactic acid, succinic acid, acetic salt and salts thereof. Frequently a mixture of buffering system is used such as sodium citrate and citric acid.

Carriers suitable for use with hair care compositions used according to the present invention include, for example, those used in the formulation of hair sprays, mousses, tonics, waters, creams gels, shampoos, conditioners, and rinses. The choice of appropriate carrier will depend on the particular product to be formulated. The carriers used herein can include a wide range of components conventionally used in hair care compositions. The carriers can contain a solvent to dissolve or disperse the styling compound being used, with water, the C₁-C₆ alcohols, lower alkyl acetate and mixtures thereof being preferred. The carriers can also contain a wide variety of additional materials such as acetone, hydrocarbons (such as isobutane, hexane, decene), halogenated hydrocarbons (such as Freons) and volatile silicones such as cyclomethicone.

When the hair care composition is a hair spray, tonic, gel, or mousse the preferred solvents include water, ethanol, volatile silicone derivatives, and mixtures thereof. The solvents used in such mixtures may be miscible or immiscible with each other. Mousses and aerosol hair sprays can also utilise any of the conventional propellants to deliver the material as a foam (in the case of a mousse) or as a fine, uniform spray (in the case of an aerosol hair spray). Further general ingredients suitable for all product forms include, sun-screening agents, anti-dandruff actives, carboxylic acid polymer thickeners for hair shampoo and conditioner compositions and emulsifiers for emulsifying the various carrier components of the compositions of the invention.

The compositions used according to the present invention may also contain adjuncts suitable for hair care. Generally such ingredients are included individually at a level of up to 2, preferably up to 1 wt% of the total composition. Suitable hair care adjuncts, include amino acids, sugars and ceramides.

The composition preferably comprises a perfume.

Compositions used according to the invention may comprise a surfactant. Surfactants are particularly useful as emulsifiers and in shampoo and conditioners.

The method of the invention comprises applying compositions as described above preferably followed by a heating step to a temperature above 50°C, more preferably above 100°C, more preferably above 180°C.

The following non-limiting Examples further illustrate the preferred embodiments of the invention. All percentages referred to in the examples and throughout this specification are by weight based on total weight unless otherwise indicated.

### Examples

### Experiment 1

25cm 2g of dark brown European wavy # 6 switches were base washed, towel dried and 0.1 gm of product described in table 1 was applied. Some switches were combed straight while others were scrunched to give a curly benefit. The switches were dried overnight and gently combed. The switches were image analysed and the length, volume etc. of the switches were obtained. The results are shown in table 2.

**Table 1**

| Combing Cream | | | | |
|---|---|---|---|---|
| **Trade Name** | **Chemical (INCI) Name** | **Supplier** | **Example A % w/w** | **Example 1 % W/W** |
| Brij 72 | Steareth-2 | Uniqema | 0.18 | 0.18 |
| Myrj 52S | PEG-40 Steareth-2 | Uniqema | 0.45 | 0.45 |
| Hydrenol MY | Cetearyl Alcohol | Cognis | 1.50 | 1.50 |
| Tinovis CD | Sodium Acrylates Copolymer, Mineral oil,PPG-1 Trideceth-6 | Ciba | 0.80 | 0.80 |
| DC 7134 | Dimethyl methylaminoethyllamine isobutyl siloxane | Dow Corning | 0.740 | 0.740 |
| DC 1788 | Dimethiconol/Dimethiconol/ Silsesquioxane Copolymer | Dow Corning | 0.800 | 0.800 |
| Estol 1517 | Isopropyl Palmitate | Croda | 1.00 | 1.00 |
| Glycerine | Glycerine | BDH | 2.00 | 2.00 |
| | Sodium titanium citrate | | 0 | 2.00 |
| | Water and minors | | To 100% | To 100% |

**Table 2**

| | Straight style (Volume in mm^2) | | | Curly Style(Volume in mm^2) | |
|---|---|---|---|---|---|
| Treatment | Example A | Example 1 | | Example A | Example 1 |
| After washing, drying and combing | 10555 | 7613 | | 11306 | 6685 |

From table 2 it is clear that switches treated with 2% sodium titanium citrate cream give lower volume straight and curly styles, than switches treated with the comparative Example.

Experiment 2 - Sensory benefits vs. water: 10 gm switches 25cm long were washed and treated with solutions containing 2% and 4% titanium citrate. Some of the switches were rinsed for 1 minute after application for 1 minute. The switches were dried at room temperature and feel tested by 18 panellists and scores were assigned on a scale of 1 to 10 on different attributes such as softness & smoothness. The scores were then normalised to control switch having a value of 5. In all cases switches treated with just water were used as control.

**Table 3: The table below gives the average of the scores of 18 panellists for each of the treatments when compared with control (water treated switches) - the control score being normalised to 5.**

| **Attribute** | **Smoothness** | | **Softness** | |
|---|---|---|---|---|
| Treatment | Average Score | Significance | Average Score | Significance |
| Control Water | 5 | | 5 | |
| 2% Ti citrate - not rinsed | 5.94 | 99.7% | 5.94 | 99.3% |
| 4% Ti citrate - not rinsed | 6.22 | 99.9% | 5.94 | 98.8% |
| 2% Ti citrate -rinsed | 5.72 | 95.1% | 5.67 | 98.2% |
| 4% Ti citrate -rinsed | 6.33 | 99.9% | 5.78 | 96.9% |

The table clearly shows that switches treated with titanium citrate are significantly better than control (p > 95% at least) for Smoothness and Softness.

### Experiment 3

Two 10 gm switches 25cm switches were washed and treated with Examples A and 1, (as described for Experiment 1) and dried at room temperature. The switches were feel tested by 18 panellists and scores were assigned on a scale of 1 to 10 on the attributes of softness and smoothness. The scores were then normalised to control switch having a value of 5.

**Table 4: The table below gives the values of the treatments with control being normalised to 5 and the significance of the difference. The values are the average scores of 18 panellists.**

| **Attribute** | **Smoothness** | | **Softness** | |
|---|---|---|---|---|
| Treatment | Average Score | Significance | Average Score | Significance |
| Example A- not rinsed | 5 | | 5 | |
| Example 1 with 2% Ti citrate - not rinsed | 5.6 | 93.1% | 6.3 | 99.9% |

The table clearly shows that the Example 1 of the invention containing 2% Ti citrate is significantly better for smoothness and softness.

### Experiment 4

Six 10 gm and 25cm switches were washed and treated with 2% Na citrate solution. Six others were washed and treated with 2% Ti citrate solutions. The switches were not rinsed. 12 Panellists were offered a pair of Na and Ti citrate solution treated switches and asked to select the switch that was better in terms of softness, smoothness and ease of comb. This was repeated for all six pairs.

**Table 5: The table below gives the number of times the two differently treated switches were selected.**

| | Number of Panellists selecting a treated switch | | |
|---|---|---|---|
| Attribute | 2% Sodium Titanium Citrate | 2% Na Citrate | Tukey-Kramer p value |
| Smoothness | 53 | 19 | 0.0001 |
| Softness | 47 | 25 | 0.0108 |
| Ease of Comb | 49 | 23 | 0.0028 |

The table clearly shows that sodium titanium citrate treated switches have significantly better sensory characteristics than Na citrate treated switches. Similar results were obtained for 4% treated switches.

### Experiment 5

25cm, 5g switches were base washed and treated by applying cream of Examples A or Example 1 (as described in Experiment 1) in a wavy line along the length of the switch (0.05g cream/g hair) and spreading it into the switch by hand taking care not to detangle the switch. The switch was massaged for 30 seconds and then left to stand for 30 seconds. The switch was held tightly in place over a wire mesh plate and rinsed under a tap horizontally in six short passes for 30 seconds. The switches were then clamped using the metal bracket and hung in place on the Instron. The rig measures the energy per stroke required to detangle the switch.

**Table 6: Table showing the values of energy required to detangle the switches. The numbers are an average of 4 switches.**

| **Treatment** | **Energy (Joules)** |
|---|---|
| Example A | 9.55 |
| Example 1 | 6.2 |

The significance (T-TEST) was found to be greater than 97%. The table clearly shows that combing cream containing 2% sodium titanium citrate required significantly less energy to detangle the switches.

## Claims

1. A method of treating hair comprising the step of applying to the hair a composition comprising from 0.001 wt% to 20 wt% of the total composition of titanium salt selected from the group consisting of citrate, oxalate and tartrate or the metal alkali salt of these and in which at least one of these is a sodium salt of titanium citrate.

2. A method of treating hair according to any preceding claim in which the level of titanium salt is from 0.01 to 10 wt%.

3. A method of treating hair according to any preceding claim which further comprises a silicone.

4. A method of treating hair according to any preceding claim which further comprises a perfume.

5. A method of treating hair according to any preceding claim which further comprises a surfactant.

6. A method of treating hair according to any preceding claim that is a leave in composition, preferably a styling composition.

7. Use of the sodium salt of titanium citrate for detangling hair.

8. Use of the sodium salt of titanium citrate for degreasing the volume of hair.

9. Use of the sodium salt of titanium citrate for smoothing hair.

10. Use of the sodium salt of titanium citrate for ease of combing.

11. Use of the sodium salt of titanium citrate for softening the hair.

## Patentansprüche

1. Verfahren zur Haarbehandlung, umfassend den Schritt des Auftragens auf das Haar einer Zusammensetzung, die 0,001 Gew.-% bis 20 Gew.-%, bezogen auf die gesamte Zusammensetzung, Titansalz umfasst, das aus der Gruppe, bestehend aus Citrat, Oxalat und Tartrat oder dem Alkalimetallsalz von diesen ausgewählt ist, und wobei wenigstens eines von diesen ein Natriumsalz von Titancitrat ist.

2. Verfahren zur Haarbehandlung gemäß einem vorangehenden Anspruch, wobei die Konzentration an Titansalz von 0,01 bis 10 Gew.-% ist.

3. Verfahren zur Haarbehandlung gemäß einem vorangehenden Anspruch, das außerdem ein Silikon umfasst.

4. Verfahren zur Haarbehandlung gemäß einem vorangehenden Anspruch, das außerdem ein Parfüm umfasst.

5. Verfahren zur Haarbehandlung gemäß einem vorangehenden Anspruch, das außerdem ein Tensid umfasst.

6. Verfahren zur Haarbehandlung gemäß einem vorangehenden Anspruch, das eine "leave-in"-Zusammensetzung, vorzugsweise eine Styling-Zusammensetzung ist.

7. Verwendung des Natriumsalzes von Titancitrat zum Entwirren von Haar.

8. Verwendung des Natriumsalzes von Titancitrat zur Verringerung des Haarvolumens.

9. Verwendung des Natriumsalzes von Titancitrat zum Glätten von Haar.

10. Verwendung des Natriumsalzes von Titancitrat zur Erleichterung des Kämmens.

11. Verwendung des Natriumsalzes von Titancitrat zum Weichmachen des Haares.

## Revendications

1. Méthode de traitement des cheveux comprenant l'étape d'application aux cheveux d'une composition comprenant de 0,001 % en poids à 20 % en poids de la composition totale de sel de titane sélectionné dans le groupe constitué du citrate, de l'oxalate et du tartrate ou du sel de métal alcalin de ceux-ci et dans laquelle au moins l'un de ceux-ci est un sel de sodium de citrate de titane.

2. Méthode de traitement des cheveux selon l'une quelconque des revendications précédentes, dans laquelle le taux de sel de titane est de 0,01 à 10 % en poids.

3. Méthode de traitement des cheveux selon l'une quelconque des revendications précédentes, comprenant en outre une silicone.

4. Méthode de traitement des cheveux selon l'une quelconque des revendications précédentes, qui comprend en outre un parfum.

5. Méthode de traitement des cheveux selon l'une quelconque des revendications précédentes, qui comprend en outre un tensioactif.

6. Méthode de traitement des cheveux selon l'une quelconque des revendications précédentes, qui est une composition sans rinçage, de préférence une composition coiffante.

7. Utilisation du sel de sodium de citrate de titane pour le démêlage des cheveux.

8. Utilisation du sel de sodium de citrate de titane pour la diminution du volume des cheveux.

9. Utilisation du sel de sodium de citrate de titane pour le lissage des cheveux.

10. Utilisation du sel de sodium de citrate de titane pour permettre de peigner facilement.

11. Utilisation du sel de sodium de citrate de titane pour l'assouplissement des cheveux.
